# EUROPÄISCHE PATENTSCHRIFT

(11) **EP 2 737 921 B1**
(45) Veröffentlichungstag und Bekanntmachung des Hinweises auf die Patenterteilung: **09.01.2019**
(21) Anmeldenummer: 13005359.8
(22) Anmeldetag: 14.11.2013
(51) Int. Cl.: A61M 16/06

(54) **Maskenwulst für ein Patienteninterface**
Mask bead for a patient interface
Jupe de masque pour une interface patient

(30) Priorität: 29.11.2012 DE 102012023268
(43) Veröffentlichungstag der Anmeldung: 04.06.2014
(60) Teilanmeldung: 18000930.0
(73) Patentinhaber: Löwenstein Medical Technology S.A., 2557 Luxembourg (LU)
(72) Erfinder: Eifler, Martin, 25348 Glückstadt (DE)
(74) Vertreter: Marx, Thomas

(56) Entgegenhaltungen:
- DE-A1-102007 057 091
- US-A1- 2008 110 464
- US-A1- 2009 145 429
- US-A1- 2010 282 265
- US-A1- 2011 174 310

## Beschreibung

Masken werden im Bereich der Beatmung und der Schlaftherapie eingesetzt, wobei ein Patient mittels einer Flow- oder Druckquelle mit Atemgas versorgt wird. Die Maske stellt die Schnittstelle zwischen Patient und Gerät dar. Sie besteht meistens aus einem Maskenkörper mit einem Maskenwulst und wird mittels einer Bänderung und evtl. einer zusätzlichen Stirnabstützung am Kopf des Patienten fixiert. Der Maskenkörper besteht in der Regel aus einem relativ steifen Kunststoff und der Maskenwulst, der am Gesicht, bzw. an Gesichtspartien des Patienten anliegt, besteht aus einem relativ dünnen, weichen Material, vorzugsweise aus einem hautfreundlichen Silikon, einem thermoplastischen Elastomer (TPE) oder aus Polyurethan und besitzt in der Regel eine Dichtlippe, um eine ausreichende Abdichtung zu gewährleisten und Leckagen zu verhindern. Herkömmliche Maskenwülste für eine Beatmungsmaske gibt es meistens üblicherweise in den Größen S, M und L, um möglichst viele verschiedene Gesichts- bzw. Nasenbeschaffenheiten der Patienten zu berücksichtigen und einen möglichst hohen Trage- und Passkomfort und eine hohe Dichtigkeit zu erzielen.

### Kritik am Stand der Technik

Derartig bekannte Lösungen haben zur Folge, dass die Anzahl der anzubietender

Masken groß ausfallen muss, um alle verschiedenen Gesichtsformen und Größen abzudecken.

US 2008/0110464 A1 D4 zeigt ein Kissen für ein Patienteninterface mit einer Basiswand, die mit einem Rahmen verbunden ist, ein darunterliegendes Stützkissen, das sich von der Basiswand in Richtung des Gesichtes erstreckt, und eine Membran, die im Wesentlichen mindestens ein Teil des darunterliegenden Kissens abdeckt. Die Membran ist angepasst, um eine kontinuierliche Abdichtung auf dem Gesicht des Patienten zu bilden. Das darunterliegende Kissen und/ oder die Basiswand definieren eine Federkonstante, die entlang einer Lange der Dichtung variiert. Dabei wird gezeigt, dass die Membrandicke in den verschiedenen Bereichen des Kissens variieren kann. Die Membran ist im Nasenrückenbereich und im oberen Wangenbereich 0,3 mm dick, im oberen Wangenbereich geht die Dicke zu 0,5 mm über und halt diese Dicke in den unteren Wangen- und Kinnbereichen aufrecht.

### Erfindung

Die vorliegende Erfindung stellt sich zur Aufgabe einen Maskenwulst für Nasalmasken und/oder Vollgesichtsmasken zu entwickeln, der eine große Anpassungsfähigkeit an verschieden große und verscheiden geformte Gesichts- und Nasenpartien der Patienten aufweist. Patienten können verschiedene Nasenformen mit hohen oder niedrigen Nasenrückenregionen, schmale oder breite sowie lange oder kurze Nasen haben. Der Maskenwulst soll deshalb in der Lage sein, sich allen möglichen Nasenformen anzupassen und die gewünschte Passform und Dichtigkeit zu erzielen.

Der Maskenwulst für ein Patienteninterface besteht aus einem Kontaktbereich zum Maskenkörper, einem dem Nasenrücken anliegenden Nasenrückenbereich mit einer Breite (B1), Seitenbereichen, dem Basisbereich - der bei einer Nasalmaske unterhalb der Nasenöffnungen auf der Oberlippe liegt und bei Vollgesichtsmasken dem Kinn anliegt - und dem Auflagebereich zum Patienten, der die eigentliche Dichtfunktion übernimmt, wobei der Auflagebereich sich ausgehend von den Seitenbereichen und dem Basisbereich sowie dem Nasenrückenbereich zur Öffnung hin erstreckt und wobei der Auflagebereich als Dichtlippe - bevorzugt eine Einzeldichtlippe - ausgeführt ist welche die zentrale Öffnung umrandet, die zumindest der Einführung der Nase des Patienten dient, wobei der Maskenwulst eine Breite (B2) aufweist und wobei das Verhältnis der Breiten B1 zu B2 eins zu drei oder größer ist. Bevorzugt ist das der Breiten B1 zu B2 eins zu zwei oder größer. Masken aus dem Stand der Technik weisen hier ein Verhältnis der Breiten B1 zu B2 von etwa eins zu vier auf.

Der erfindungsgemäße Maskenwulst ist auch dadurch gekennzeichnet, dass im Nasenrückenbereich eine Wandstärke (W1) von 0,6 mm verwendet wird.

Der erfindungsgemäße Maskenwulst ist auch dadurch gekennzeichnet, dass der Basisbereich, mit der Wandstärke (W3), im Gegensatz zu den Seitenbereichen, mit den Wandstärken (W2), ebenfalls dünn ausgeführt ist, der Basisbereich eine Wandstärke (W3) im

Bereich 0,8 - 1,2 mm aufweist und/oder dass der Seitenbereich eine Wandstärke (W2) im Bereich 1,5 - 2,8 mm aufweist, und wobei der Auflagebereich eine annähernd gleichbleibende Wandstärke (W4) im Bereich 0,2 - 0,75 mm aufweist.

Der erfindungsgemäße Maskenwulst ist auch dadurch gekennzeichnet, dass der Nasenrückenbereich eine Breite (B1) im Bereich 27 - 37 mm aufweist.

Der erfindungsgemäße Maskenwulst ist auch dadurch gekennzeichnet, dass die Höhe (H1) im Nasenrückenbereich im Bereich 18 - 27 mm, bevorzugt 20 - 25 mm ist.

Der erfindungsgemäße Maskenwulst ist auch dadurch gekennzeichnet, dass die Höhe (H4) im Nasenrückenbereich im Bereich 14 - 23 mm ist.

Der erfindungsgemäße Maskenwulst ist auch dadurch gekennzeichnet, dass der Nasenrückenbereich eine bogenförmige Kontur (R), zum Beispiel mit einem Winkel □ im Bereich größer als 90°, aufweist.

Ein beispielhafter Maskenwulst zeichnet sich dadurch aus, dass die Höhe (H3) des Basisbereiches im Bereich 16 - 21 mm ist.

Ein beispielhafter Maskenwulst zeichnet sich dadurch aus, dass die Höhe (H6) des Basisbereiches im Bereich 8,5 - 13,5 mm ist.

Ein beispielhafter Maskenwulst zeichnet sich dadurch aus, dass die Höhe (H2) des Seitenbereiches im Bereich 12 - 22 mm, bevorzugt im Bereich 14 - 16 mm ist.

Ein beispielhafter Maskenwulst zeichnet sich dadurch aus, dass die Höhe (H5) des Seitenbereiches im Bereich 6 - 14 mm ist. Der erfindungsgemäße Maskenwulst ist auch dadurch gekennzeichnet, dass die Wanddicke im Seitenbereich von der Basis (W2) hin zum Auflagebereich (W4) hin kontinuierlich abnimmt.

Der erfindungsgemäße Maskenwulst ist auch dadurch gekennzeichnet, dass das Verhältnis von Länge (L) des Maskenwulstes zu Breite (B2) ungefähr 1 zu 1 ist.

Ein beispielhafter Maskenwulst zeichnet sich dadurch aus, dass die Breite (B2) etwa im Größenbereich 54 mm bis 65 mm ist.

Der erfindungsgemäße Maskenwulst ist auch dadurch gekennzeichnet, dass
der Auflagebereich an den Übergängen Nasenrückenbereich zum Seitenbereich und Basisbereich zum Seitenbereich Eckpunkte (E1 - E4) aufweist die der Ausrichtung der Maske auf dem Gesicht dienen.

Der erfindungsgemäße Maskenwulst ist auch dadurch gekennzeichnet, dass die unteren Eckpunkte (E3, E4) sich beiderseits der Symmetrieebene (x-x) befinden und der Ausrichtung des Wulstes an der Nasolabial-Falte dienen.

Der erfindungsgemäße Maskenwulst ist auch dadurch gekennzeichnet, dass die oberen Eckpunkte (E1, E2) seitlich der Nase im Bereich liegen und das Ende des Stützbereiches und somit den Übergang zum Nasenrückenbereich mit der Breite (B1) definieren.

Der erfindungsgemäße Maskenwulst ist auch dadurch gekennzeichnet, dass das Material des Maskenwustes eine Dehnbarkeit aufweist, die über 800 % liegt.

Ein beispielhafter Maskenwulst zeichnet sich dadurch aus dass das der Wulst im 2K Verfahren hergestellt ist.

Um einen großen Bereich an unterschiedlichsten Nasengrößen und - formen abzudecken, wird ein Maskenwulst vorgeschlagen, der von der Länge im Größenbereich einer Maske der Größe S und von der Breite im Größenbereich einer Maske der Größe M liegt. Daraus ergibt sich ein Verhältnis von Länge (L) zu Breite (B2) des Maskenwulstes welches ungefähr 1:1 ist. Zum Anknüpfen an den Maskenkörper dient beispielsweise ein umlaufendes u-förmiges Halteprofil als Kontaktbereich, welches einen Innenschenkel und einen Außenschenkel mit einem Hinterschnitt aufweist und über einer umlaufenden Kante am Maskenkörper einrastet. Zur genauen Positionierung dienen Querstege im Halteprofil des Maskenwulstes, die in Nuten im Maskenkörper eingefügt werden müssen. Möglich ist es aber auch, den Maskenwulst im Kontaktbereich mittels 2K-Verfahren mit dem Maskenkörper zu verbinden.

Um eine hohe Anpassungsfähigkeit an verschieden große und verschieden geformte Nasenpartien eines Patienten zu erzielen, weist der Maskenwulst im Nasenrückenbereich eine sehr breite Form (B1) mit einem sehr großen, dünnwandigen Bereich auf, der sich leicht verformen lässt.

Die Anpassungsfähigkeit an verschieden große und verschieden geformte Nasenpartien der Patienten zeigt sich insbesondere in der besonderen Ausführung des Nasenrückenbereichs. Aus dem Vergleich der Erfindung mit dem Stand der Technik wird deutlich, dass bei dem erfindungsgemäßen Wulst neben dem Verhältnis B1 zu B2 auch das Verhältnis H1 zu H2 vom Stand der Technik abweicht. Erfindungsgemäß ist H1 immer gleichgroß oder größer als H2. Bei Masken aus dem Stand der Technik ist H1 immer kleiner als H2.

Die Erfindung ist anwendbar für elastische Maskenwülste, die auf einen harten Maskenkörper lösbar oder dauerhaft befestigt sind, und ebenso für Maskenwülste, die vollständig aus einem elastischen Material gefertigt sind. Maskenwülste, die vollständig aus einem elastischen Material gefertigt sind, weisen dann statt dem harten Maskenkörper entweder einen harten Maskenrahmen auf, in den der Maskenwulst eingefügt ist, oder weisen einen verdickten oder versteiften Abschnitt auf, die die Stützfunktion des harten Maskenkörpers übernimmt. Der Kontaktbereich zum Maskenkörper ist dann regelmäßig die Region, wo der elastische Maskenwulst in einen verdickten oder versteiften Abschnitt übergeht oder von dem Maskenrahmen gestützt wird.

Der Maskenwulst ist aus einem angenehmen hautfreundlichen Material, bevorzugt aus Silikon in einem Shore-Härtebereich zwischen 35 bis 55 Shore A, bevorzugt 40 - 50 Shore A.

Der Maskenwulst kann im 2K-Verfahren hergestellt sein, wobei der Wulst aus einem weichen Material hergestellt wird und bevorzugt an ein hartes Material angespritzt wird. Das harte Material definiert den Kontaktbereich oder der Maskenkörper oder ein Teil des Wulstes mit Stützfunktion. Im letztgenannten Fall ist der Wulst zweiteilig aus einem weichen und einem harten Material hergestellt. Der Kontakt wird bevorzugt über eine Haftung der Materialien erreicht, alternativ oder zusätzlich können mechanische Kontaktstellen die Haftung verstärken (Formschluss).

Ein beispielhafter Wulst kann auch einen Hohlraum aufweisen, der ein federnd nachgiebiges Füllmaterial aufweist. Der Hohlraum ist bevorzugt umlaufend ausgestaltet, in einer alternativen Ausführungsform ist im Nasenrückenbereich kein gefüllter Hohlraum vorgesehen. Das Füllmaterial kann ein Gel oder Schaum oder Gas oder eine Flüssigkeit oder ein weicher Füllstoff sein. Ein Gel würde im flüssigen Zustand in den Hohlraum eingefüllt werden und dort aushärten. Das Füllmaterial weist bevorzugt eine Härte im Bereich 2 Shore 000 bis 20 Shore 00, bevorzugt im Bereich 2 Shore 00 bis 10 Shore 00, besonders bevorzugt im Bereich 10 Shore 00 bis 20 Shore 00. Die Füllung kann auch unterschiedlich hart ausgeführt werden. Gesichtsnah weicher und Gesichtsfern härter. Der Maskenwust ist bevorzugt aus Silikon oder PU hergestellt. Das Material des Maskenwustes weist eine Dehnbarkeit auf, die im Bereich über 500 % liegt, bevorzugt bei über 800 %, besonders bevorzugt über 1000 %.

### Figurenbeschreibung

- Fig.1a:: Maskenwulst (M) in Draufsicht
- Fig.1b:: Maskenwulst (M) in Seitenansicht
- Fig.1c:: Maskenwulst (M) von oben

- Fig.2a:: vertikale Schnittdarstellung des Maskenwulstes (M) entlang der Ebene x/x
- Fig.2b:: horizontale Schnittdarstellung des Maskenwulstes (M) entlang der Ebene y/y
- Fig.2c:: horizontale Schnittdarstellung des Maskenwulstes (M) entlang der Ebene z/z

- Fig.3:: Vergleich Erfindung zum Stand der Technik Perspektivdarstellungen von Maskenwülsten (M),(M') und (M")
- Fig.4:: Maskenwulst (M') in verschiedenen Ansichten
- Fig.5:: Schnittdarstellungen des Maskenwulstes (M')
- Fig.6:: Maskenwulst (M") in verschiedenen Ansichten
- Fig.7:: Schnittdarstellungen des Maskenwulstes (M")

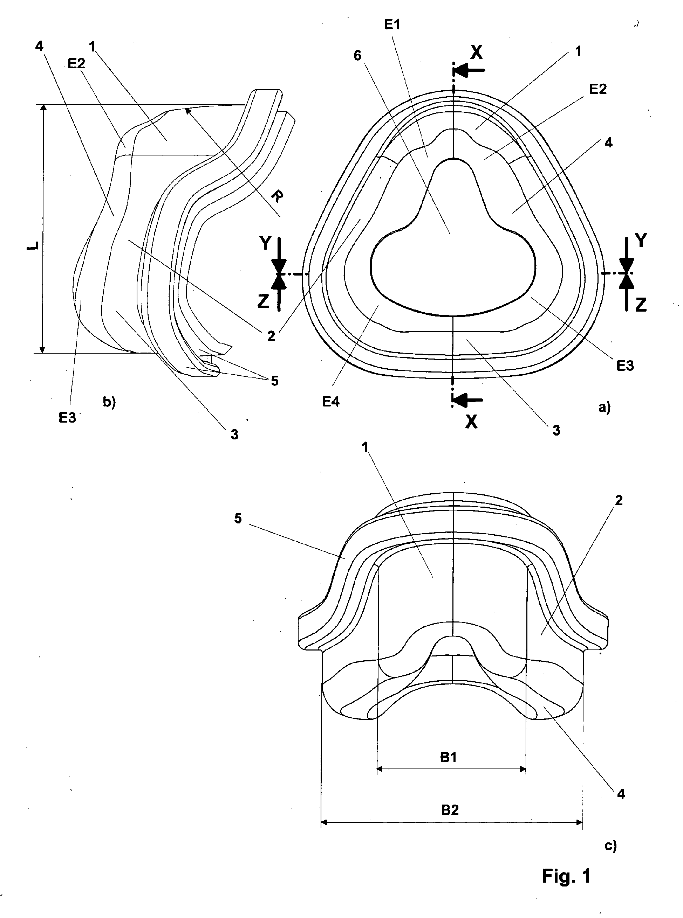

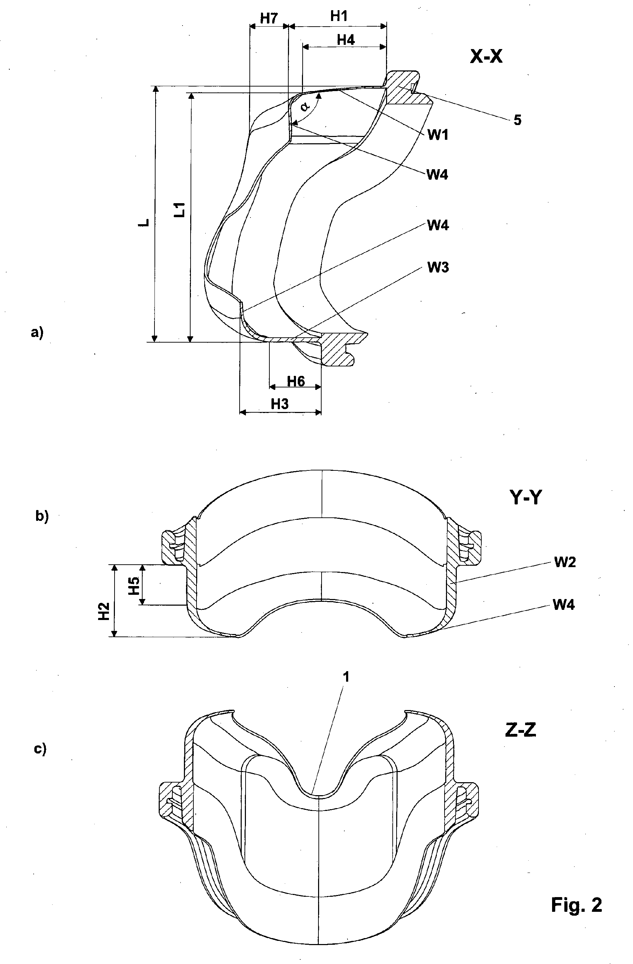

Die Figur 1a zeigt den Maskenwulst in der Draufsicht. Eingezeichnet ist eine vertikale Schnittebene x und eine horizontale Schnittebene y/z. Die Schnitte sind in Figur 2 veranschaulicht. Der Maskenwulst weist einen Nasenrückenbereich (1), Seitenbereiche (2), den Basisbereich (3) und den Auflagebereich (4) zum Patienten auf. Die Seitenbereiche (2) erstrecken sich ausgehend vom Kontaktbereich zum Maskenkörper (5) im Wesentlichen vertikal nach oben. Der Auflagebereich (4) zum Patienten ist als eine Einzeldichtlippe ausgeführt, die den anderen Bereichen angeformt ist und innen eine Öffnung (6) freigibt, die der Einführung zumindest der Nase des Patienten dient. Der Auflagebereich (4) erstreckt sich ausgehend von den Seitenbereichen (2) und dem Basisbereich (3) sowie dem Nasenrückenbereich (1) zur Öffnung hin gebogen, wobei sich der Auflagebereich (4) zumindest teilweise horizontal zum Seitenbereich (2) erstreckt.

Die Figur 1b zeigt den Maskenwulst in der Seitenansicht. Im Nasenrückenbereich (1) ist der Patient am empfindlichsten, daher wird hier eine sehr dünne Wand (W1) von ≤ 0,6 mm, bevorzugt im Bereich 0,25 - 0,5 mm gewählt. Der Nasenrückenbereich lässt sich leicht verformen und der Nasenform anpassen. Um ein Einknicken dieses Bereiches bei Druck auf den Bereich zu vermeiden, wurde in Gegensatz zu den Bereichen (2 und 3), die eine gerade Kontur aufweisen, eine bogenförmige Kontur (R) gewählt. Der Bogen im Nasenrückenbereich erstreckt sich vom Kontaktbereich zum Maskenkörper (5) hin zur Auflagefläche (4). Möglich ist aber auch, die Kontur (R) linear verlaufen zu lassen.

In Figur 1b ist ebenfalls zu erkennen, dass der Wulst eine Kontaktstelle zum Patientengesicht im Bereich des dünnwandigen Nasenrückenbereiches aufweist und eine dünnwandige Oberlippenauflage die durch einen seitlichen Stützbereich verbunden sind. An den Übergängen ergeben sich dominante Eckpunkte die der Ausrichtung der Maske auf dem Gesicht dienen.

Die unteren Eckpunkte (E3, E4) befinden sich beiderseits der Symmetrieebene und dienen bevorzugt der Ausrichtung der Maske an der Nasolabial Falte. Die oberen Eckpunkte (E1, E2) liegen seitlich der Nase (im Bereich der Maxilla neben dem Nasenbein) und definieren das Ende des Stützbereiches und somit den Übergang zum Nasenrückenbereich mit der Breite (B1).

Figur 1c zeigt den Maskenwulst in der Ansicht von oben. Die Breite des Nasenrückenbereiches (B1) wurde breiter als bei bekannten Masken gewählt, um eine optimale Passform und Dichtigkeit auch bei breiteren und auch flachen Nasenformen zu gewährleisten. Durch die gewählte Geometrie sitzt der Maskenwulst tiefer auf der Nase des Anwenders. Ein hoher Nasenrücken taucht bei der gewählten Kontur tiefer in den Maskenwulst ein, was aber keinen Nachteil darstellt.

Um einen großen Bereich an unterschiedlichsten Nasengrößen und - formen abzudecken, wird ein Maskenwulst mit einer Länge (L) im Größenbereich von 50 mm bis 65 mm, bevorzugt 53 bis 62 mm, besonders bevorzugt 58 mm, und von einer Breite (B2) im Größenbereich 50 mm bis 65 mm, bevorzugt 53 bis 63 mm, besonders bevorzugt 59 mm, vorgeschlagen. Daraus ergibt sich ein Verhältnis von Länge (L) zu Breite (B2) von ungefähr 1:1.

Die Länge (L) liegt im Größenbereich einer handelsüblichen Maske der Größe S, wogegen die Breite (B2) im Größenbereich einer Maske der Größe M liegt. Die gesamten Proportionen der Maske sind dadurch im Vergleich zum Stand der Technik verändert worden.

Der Nasenrückenbereich (1) ist im Vergleich zum Stand der Technik wesentlich breiter, die Breite (B1) ist im Bereich 27 - 37 mm, bevorzugt bei 31 bis 35 mm, besonders bevorzugt bei 32,5 bis 35 mm. Der Nasenrückenbereich (1) kennzeichnet sich bevorzugt durch einen ersten Radius starker, bevorzugt konstanter, Krümmung, der im Seitenbereich in einen zweiten Radius mit weniger ausgeprägter Krümmung oder einen im Wesentlichen geraden Bereich (ohne Krümmung) übergeht. Bevorzugt ist die Krümmung im Seitenbereich halb so groß oder kleiner als im Nasenrückenbereich. Der Nasenrückenbereich (1) ist im Vergleich zum Stand der Technik dünnwandiger mit einer Wandstärke (W1) ≤ 0,6 mm, bevorzugt 0,25 bis 0,5 mm.

Die Figur 2a zeigt den Wulst in der Ebene x geschnitten. Im Nasenrückenbereich (1) ist der Patient am empfindlichsten, daher wird hier eine sehr dünne Wand (W1) von ≤ 0,6 mm, bevorzugt 0,2 - 0,5 mm, besonders bevorzugt 0,25 mm gewählt. Dieser Bereich lässt sich leicht verformen und der Nasenform anpassen. Um ein Einknicken dieses Bereiches bei Druck auf den Bereich zu vermeiden, wurde in Gegensatz zu den Bereichen (2 und 3), die eine gerade Kontur aufweisen, eine bogenförmige Kontur (R), vom Kontaktbereich zum Maskenkörper (5) hin zur Auflagefläche (4) im Nasenrückenbereich, gewählt. Der Winkel □□□gemessen von der Vertikalen (hier durch W4 veranschaulicht) zur Oberfläche der bogenförmigen Kontur (R), liegt daher bevorzugt im Bereich größer als 90°, besonders bevorzugt bei 91° bis 100°. Möglich ist aber auch die Kontur (R) linear verlaufen zu lassen, der Winkel □ ist dann 90°.

Der Basisbereich (3), mit der Wandstärke (W3), ist im Gegensatz zu den Seitenbereichen (2), mit den Wandstärken (W2), ebenfalls dünn ausgeführt, da ein Druck auf den Kiefer als unangenehm empfunden wird. Die Wandstärke (W3) beträgt im Bereich 0,8 - 1,2 mm, bevorzugt 1,0 mm. Da der Patient an den Kontaktstellen im Seitenbereich (2) des Maskenwulstes am unempfindlichsten ist, können die Wandstärken (W2) dieses Bereiches dicker ausgeführt sein und sorgen so für die Steifigkeit und Stützkraft des Maskenwulstes. Die Wandstärke (W2) beträgt in diesem Bereich 1,5 - 2,8 mm, bevorzugt 2,1 mm. Der Auflagebereich (4) zum Patienten ist als eine Einzeldichtlippe ausgeführt. Der Auflagebereich (4) wurde auf dem kompletten Umfang mit einer annähernd gleichbleibenden, dünnen Wandstärke (W4) von 0,2 - 0,75 mm, bevorzugt 0,5 mm, ausgebildet, die dem Patienten einen angenehmen Tragekomfort ermöglicht und zudem als Dichtlippe dient.

Maskenwülste, die aus dem Stand der Technik bekannt sind, weisen eine Doppellippe auf, wobei die innere Lippe als Stützlippe und die äußere Lippe als Dichtlippe ausgeführt ist. Die Wandstärken der einzelnen Bereiche (1 - 4) wurden erfindungsgemäß auf die Empfindlichkeit der Kontaktstellen im Gesicht des Anwenders angepasst. Durch die erfindungsmäßige Wahl von unterschiedlichen Wandstärken (W1-W4) der Bereiche (1-4) des Maskenwulstes kann auf eine zusätzliche Stützlippe verzichtet werden.

Die Höhe (H1) im Nasenrückenbereich beträgt 18 - 27 mm, bevorzugt 20 - 25 mm, besonders bevorzugt 22,2 mm und ist höher als bei Masken aus dem Stand der Technik. Dadurch wird ein weiter Bereich mit guten Dichteigenschaften für unterschiedliche Nasenrücken eröffnet.

Die Höhe (H4) im Nasenrückenbereich ist höher als bei handelsüblichen Maskenwülsten und beträgt im Bereich 14 - 23 mm, bevorzugt 18,9 mm.

Die Höhe (H3) des Basisbereiches (3) ist geringer als bei handelsüblichen Maskenwülsten und beträgt im Bereich 16 - 21 mm, bevorzugt 18,5 mm.

Die Höhe (H6) des Basisbereiches (3) ist ebenfalls geringer als bei handelsüblichen Maskenwülsten und beträgt im Bereich 8,5 - 13,5 mm, bevorzugt 11,9 mm und hat eine Stützfunktion. Die Höhe (H7) (Differenz zwischen der Nasenrückenauflage und der Auflage im Seitenbereich) entspricht im Wesentlichen den Höhen der Masken aus dem Stand der Technik.

Die Figur 2b zeigt den Wulst in der Ebene y geschnitten. Die Höhe (H2) des Seitenbereiches (2) ist geringer als bei handelsüblichen Maskenwülsten und beträgt im Bereich 12 - 22 mm, bevorzugt 15,8 mm. Die Höhe (H5) des Seitenbereiches (2) ist geringer als bei handelsüblichen Maskenwülsten und beträgt im Bereich 6 - 14 mm, bevorzugt 8,4 mm. Zur erkennen ist zudem, wie die Wanddicke im Seitenbereich von der Basis (W2) hin zum Auflagebereich (W4) kontinuierlich abnimmt.

Die Figur 2c zeigt den Wulst in der Ebene z geschnitten. Hier erkennt man gut, dass der Nasenrückenbereich (1) vertieft ist.

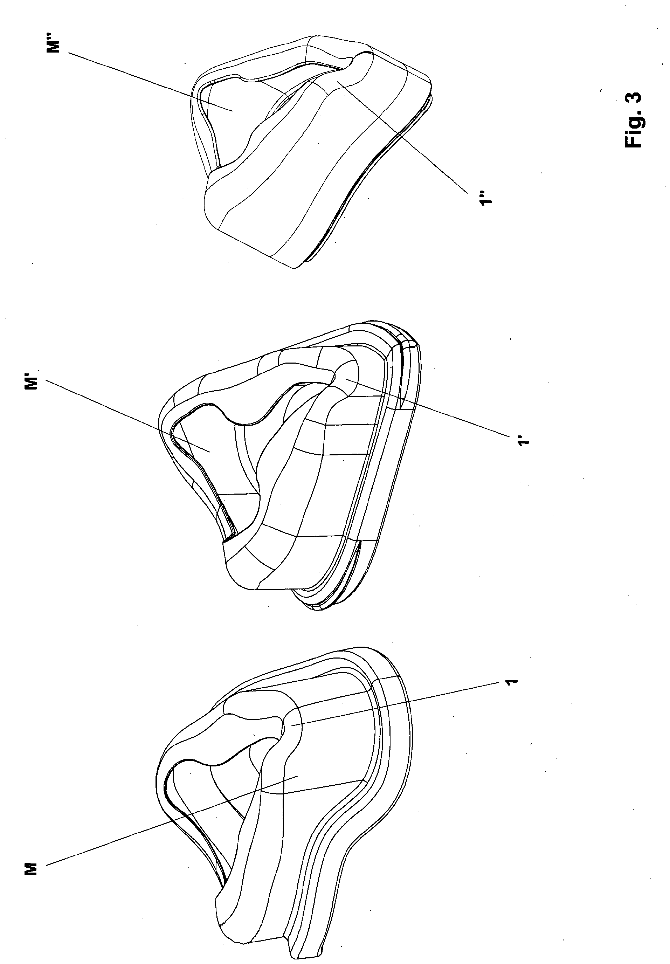

In der Figur 3 werden die Unterscheide des erfindungsmäßigen Maskenwulstes (M) zu Maskenwülsten aus dem Stand der Technik (M') und (M") in der Perspektivdarstellung deutlich. Insbesondere ist gut erkennbar, dass der Nasenrückenbereich (1) höher ist als die Nasenrückenbereiche (1'und 1").

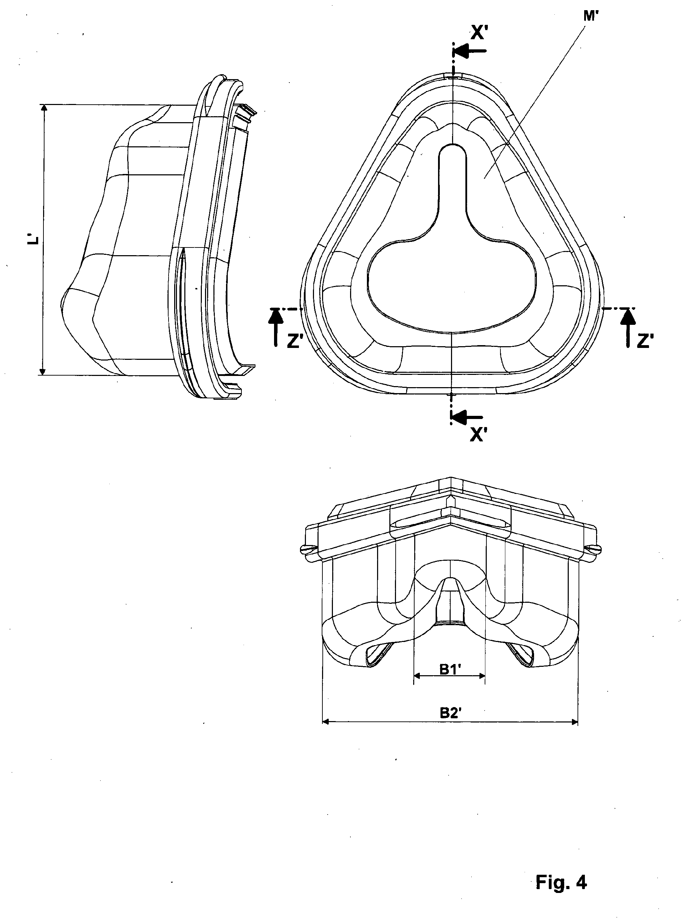

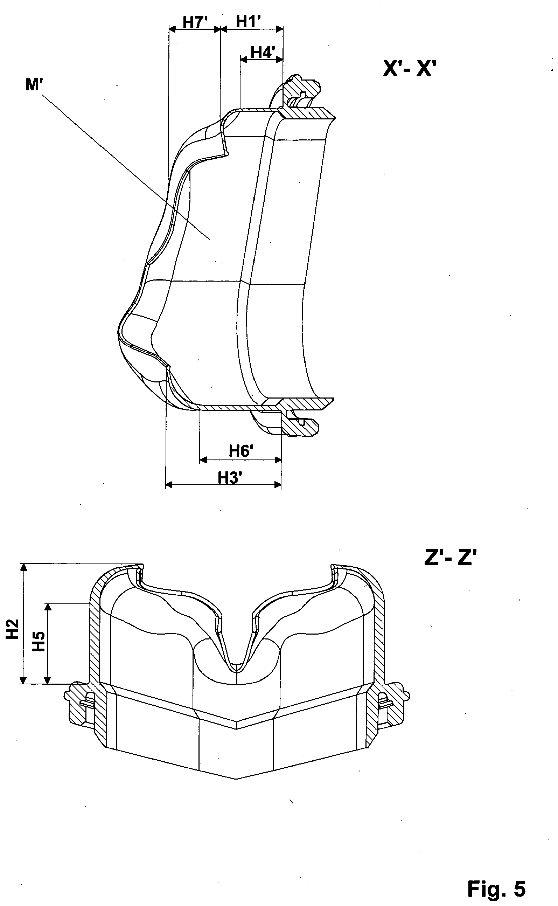

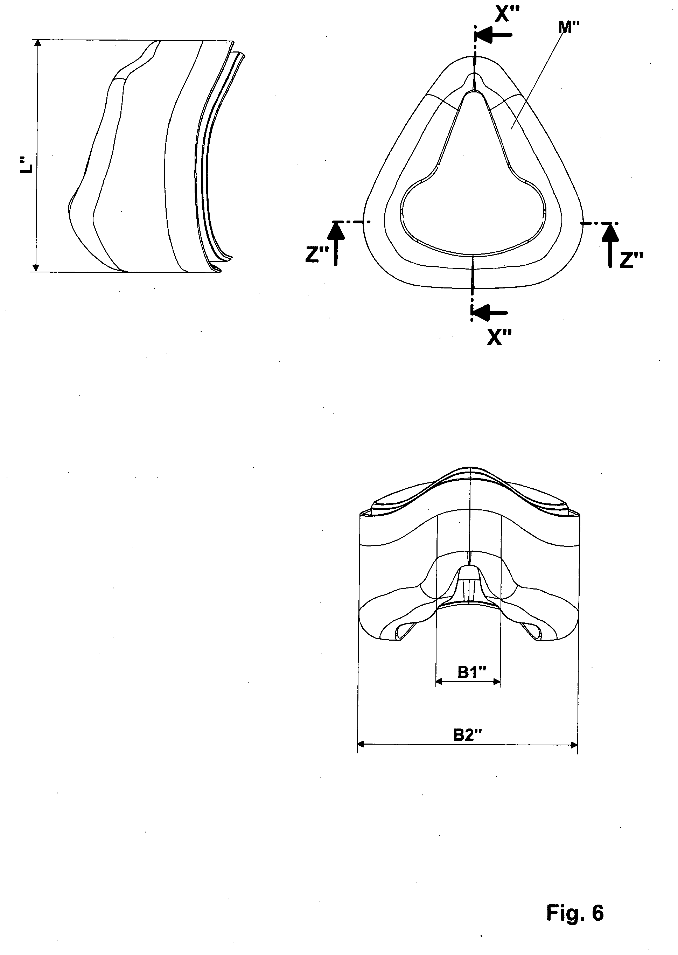

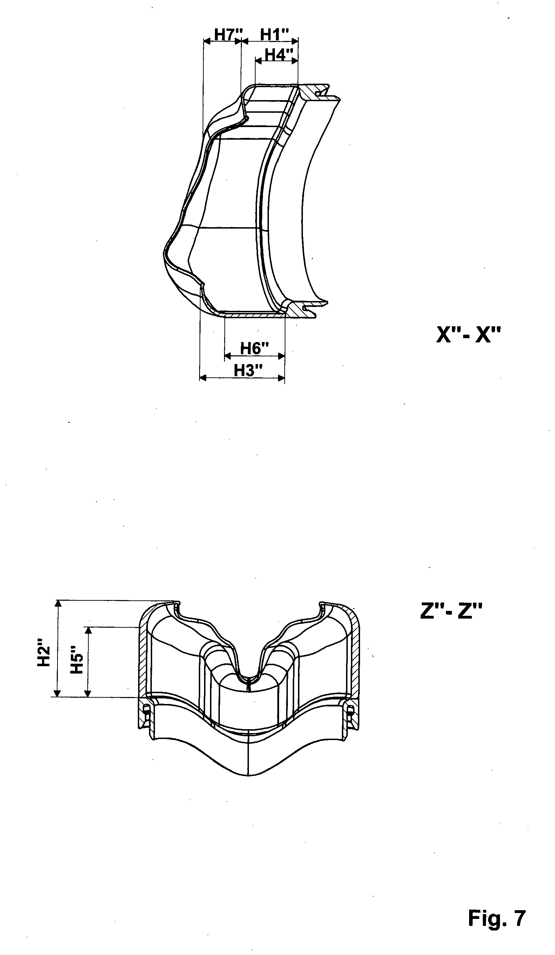

Die Figuren 4 und 5 zeigen einem Maskenwulst (M') und die Figuren 6 und 7 einen weiteren Maskenwulst (M") aus dem Stand der Technik.

Eine Gegenüberstellung der Masse des Maskenwulstes (M) zu den Massen der Maskenwülste (M' und M") macht den Unterscheid der Erfindung zum Stand der Technik deutlich.

Das maßgebliche Merkmal des erfindungsgemäßen Maskenwulstes (M) ist der sehr große Verformbereich des Nasenrückenbereiches (1), der durch die geeignete Wahl der Wandstärke (W1) und der Breite (B1) und der ersten Höhe (H1) und der weiteren Höhe (H4) erzielt wird. Dieser Bereich ist ausschlaggebend für die Anpassungsfähigkeit an verschieden große und verschieden geformte Nasenpartien der Patienten.

Die Breite (B2) ist die maximale Breite des Maskenwulstes gemessen an den Außenkanten der Seitenbereichen (2) im Bereich neben der Kontaktstelle (5) zum Maskenkörper. Die Breite (B2) ist alternativ die maximale Breite des Kontaktbereiches.

Die Länge (L) ist die maximale Länge des Maskenwulstes gemessen in der Symmetrieebene der Maske zwischen Nasenrückenbereich und Oberlippenbereich.

Die Anpassungsfähigkeit an verschieden große und verschieden geformte Nasenpartien der Patienten zeigt sich insbesondere in der besonderen Ausführung des Nasenrückenbereichs. Aus dem Vergleich der Erfindung mit dem Stand der Technik (siehe Figuren 3 bis 7) wird deutlich, dass bei dem erfindungsgemäßen Wulst neben dem Verhältnis B1 zu B2 auch das Verhältnis H1 zu H2 vom Stand der Technik abweicht. Erfindungsgemäß ist H1 immer gleichgroß oder größer als H2. Bei Masken aus dem Stand der Technik ist H1 immer kleiner als H2.

Die Länge (L) beträgt bevorzugt 58 mm und ist im Vergleich zu den Längen (L') mit 61,5 mm und (L") mit 62,5 mm kürzer.

Die Breite (B2, B2' und B2") ist bei allen drei Wülsten gleich und beträgt im Bereich 59 mm; wohingegen die Breite des Nasenrückenbereiches (B1) im Verhältnis zu den Breiten (B1') und (Bl") doppelt so breit ist und erfindungsgemäß im Bereich 33,7 mm beträgt. Die Wandstärke (W1) ist im Vergleich zu den Wandstärken (W1') und (W1") halb so dick und liegt im Bereich zwischen 0,25 mm und 0,5 mm. Die Höhe (H1) beträgt im Bereich 18 - 27 mm, hier 22,2 mm und ist im Vergleich zu den Höhen H1'=12,7 mm und H1"=15,2 mm, um etwa das 1,4- bis 2,1-fache höher.

Die Höhe (H2) der Seitenbereiche (2) beträgt etwa nur das 0,6fache der Höhen (H2' und H2"), die Höhe (H3) des Kinnbereiches (3) beträgt etwa das 0,7fache der Höhen (H3' und H3") und ist somit kleiner als bei handelsüblichen Maskenwülsten.

Die Höhe (H7) (Differenz zwischen der Nasenrückenauflage und der Auflage im Seitenbereich) ist in etwa gleich den Höhen (H7' und H7").

Um eine hohe Anpassungsfähigkeit an verschieden große und verschieden geformte Nasenpartien eines Patienten zu erzielen, weist der Maskenwulst im Nasenrückenbereich eine sehr breite Form (B1) mit einem sehr großen, dünnwandigen Bereich auf, der sich leicht verformen lässt. Der Maskenwulst weist in diesem Bereich, der etwa 1/3 des Umfanges ausmacht, eine Wandstärke von ≤ 0,6 mm auf und ist in diesem Bereich zudem um etwa das 2-fache höher als in den Seitenbereichen des Wulstes. Um aber ein Einknicken dieses Bereiches bei Druck auf den Bereich zu vermeiden, wurde eine bogenförmige Kontur vom Kontaktbereich hin zur Auflagefläche im Nasenrückenbereich gewählt.

Die Erfindung eignet sich besonders für Masken die nur die Nase des Anwenders überdecken; obwohl auch gute Ergebnisse mit erfindungsgemäßen Vollgesichtsmasken erzielt wurden, die Mund und Nase des Anwenders überdecken.

Der Maskenwulst weist einen Bereich auf der sich zu Aufbringung einer Kennzeichnung eignet. Bevorzugt ist der Bereich im Oberlippenbereich. Dieser kann beispielsweise bedruckt oder gelasert oder graviert sein.

## Patentansprüche

1. Maskenwulst für ein Patienteninterface bestehend aus einem Kontaktbereich, einem Nasenrückenbereich (1), Seitenbereichen (2), einem Basisbereich (3) und einem Auflagebereich (4) zum Patienten, wobei der Auflagebereich (4) sich ausgehend von den Seitenbereichen (2) und dem Basisbereich (3) sowie dem Nasenrückenbereich (1) zu einer Öffnung (6) hin erstreckt, wobei der Auflagebereich (4) als Dichtlippe ausgeführt ist, welche die zentrale Öffnung (6) umrandet, die zumindest der Einführung der Nase des Patienten dient, wobei der Nasenrückenbereich (1) eine Breite (B1), im Übergangsbereich vom Nasenrückenbereich (1) zum Seitenbereich (2), aufweist, der Maskenwulst eine maximale Breite (B2) aufweist und das Verhältnis der Breiten (B1) zu (B2) eins zu drei oder größer ist und wobei im Nasenrückenbereich (1) eine Wandstärke (W1) maximal 0,6 mm beträgt, **dadurch gekennzeichnet, dass** der Basisbereich (3), der eine Wandstärke (W3) aufweist, im Gegensatz zu den Seitenbereichen (2), die eine Wandstärke (W2) aufweisen, ebenfalls dünn ausgeführt ist, wobei der Basisbereich (3) eine Wandstärke (W3) im Bereich 0,8 - 1,2 mm aufweist und/oder dass der Seitenbereich (2) eine Wandstärke (W2) im Bereich 1,5 - 2,8 mm aufweist, und wobei der Auflagebereich (4) eine annähernd gleichbleibende Wandstärke (W4) im Bereich 0,2 - 0,75 mm aufweist.

2. Maskenwulst nach Anspruch 1 **dadurch gekennzeichnet, dass** der Nasenrückenbereich (1) eine Breite (B1) im Bereich 27 - 37 mm aufweist.

3. Maskenwulst nach zumindest einem der vorhergehenden Ansprüche **dadurch gekennzeichnet, dass** die Höhe (H1) im Nasenrückenbereich, gemessen von der Kontaktstelle (5) zum Maskenkörper bis zum Auflagebereich (4) im Bereich 18 - 27 mm ist.

4. Maskenwulst nach zumindest einem der vorhergehenden Ansprüche **dadurch gekennzeichnet, dass** die Höhe (H4) im Nasenrückenbereich, gemessen von der Kontaktstelle (5) zum Maskenkörper bis zum Übergang der Nasenrückbereich (1) zum Auflagebereich (4) im Bereich 14 - 23 mm ist.

5. Maskenwulst nach zumindest einem der vorhergehenden Ansprüche **dadurch gekennzeichnet, dass** der Nasenrückenbereich (1) eine bogenförmige Kontur (R) aufweist.

6. Maskenwulst nach zumindest einem der vorhergehenden Ansprüche **dadurch gekennzeichnet, dass** die Wanddicke im Seitenbereich von der Basis hin zum Auflagebereich (4) hin kontinuierlich abnimmt.

7. Maskenwulst nach zumindest einem der vorhergehenden Ansprüche **dadurch gekennzeichnet, dass** das Verhältnis von Länge (L) des Maskenwulstes, gemessen von der Kontaktstelle (5) zum Nasenrückenbereich (1) bis zu der Kontaktstelle (5) zum Basisbereich (3), in der Symmetrieebene X-X der Maske zwischen Nasenrückenbereich und Oberlippenbereich, zu Breite (B2) ungefähr 1 zu 1 ist.

8. Maskenwulst nach zumindest einem der vorhergehenden Ansprüche **dadurch gekennzeichnet, dass** die Höhe (H1) im Nasenrückenbereich gemessen von der der Kontaktstelle (5) zum Maskenkörper bis zum Auflagebereich (4) immer gleichgroß oder größer als die Höhe (H2) im Seitenbereich gemessen von der Kontaktstelle (5) zum Maskenkörper bis zum Auflagebereich (4) ist.

9. Maskenwulst für ein Patienteninterface nach zumindest einem der vorhergehenden Ansprüche, **dadurch gekennzeichnet, dass** der Auflagebereich (4) an den Übergängen Nasenrückenbereich (1) zum Seitenbereich (2) und Basisbereich (3) zum Seitenbereich (2) Eckpunkte (E1 - E4) aufweist die der Ausrichtung der Maske auf dem Gesicht dienen.

10. Maskenwulst nach Anspruch 9 **dadurch gekennzeichnet, dass** die unteren Eckpunkte (E3, E4) sich beiderseits der Symmetrieebene (x-x) befinden und der Ausrichtung des Wulstes an der Nasolabial-Falte dienen.

11. Maskenwulst nach zumindest einem der Ansprüche 9 oder 10 **dadurch gekennzeichnet, dass** die oberen Eckpunkte (E1, E2) seitlich der Nase im Bereich liegen und das Ende des Stützbereiches und somit den Übergang zum Nasenrückenbereich mit der Breite (B1) definieren.

12. Maskenwulst nach zumindest einem der vorhergehenden Ansprüche **dadurch gekennzeichnet, dass** das Material des Maskenwustes eine Dehnbarkeit aufweist, die über 800 % liegt.

## Claims

1. A mask bead for a patient interface consisting of a contact region, a nasal bridge region (1), lateral regions (2), a base region (3) and a contact region (4) for the patient, wherein the contact region (4) extends from the lateral regions (2) and the base region (3) as well as the nasal bridge region (2) toward an opening (6), wherein the contact region (4) is designed as a sealing lip that surrounds the central opening (6) which at least serves to introduce the nose of the patient, wherein the nasal bridge region (1) has a width (B1) at the transition region from the nasal bridge region (1) to the lateral region (2), the mask bead has a maximum width (B2), and the ratio of the widths (B1) to (B2) is one to three or greater, and wherein a wall thickness (W1) is at a maximum 0.6 mm in the nasal bridge region (1), **characterized in that** the base region (3) that has a wall thickness (W3) is also designed thin in contrast to the lateral regions (2) that have a wall thickness (W2), wherein the base region (3) has a wall thickness (W3) within a range of 0.8-1.2 mm, and/or the lateral region (2) has a wall thickness (W2) within a range of 1.5-2.8 mm, and wherein the contact region (4) has a nearly constant wall thickness (W4) within a range of 0.2-0.75 mm.

2. The mask bead according to claim 1, **characterized in that** the nasal bridge region (1) has a width (B1) within a range of 27-37 mm.

3. The mask bead according to at least one of the preceding claims, **characterized in that** the height (H1) in the nasal bridge region is within a range of 18-27 mm measured from the contact point (5) of the mask body up to the contact region (4).

4. The mask bead according to at least one of the preceding claims, **characterized in that** the height (H4) in the nasal bridge region is within a range of 14-23 mm measured from the contact point (5) of the mask body up to the transition to the nasal bridge region (1) of the contact region (4).

5. The mask bead according to at least one of the preceding claims, **characterized in that** the nasal bridge region (1) has an arc-shaped contour (R).

6. The mask bead according to at least one of the preceding claims, **characterized in that** the wall thickness in the lateral region decreases continuously from the base to the contact region (4).

7. The mask bead according to at least one of the preceding claims, **characterized in that** the ratio is about 1 to 1 between the length (L) of the mask bead, measured from the contact point (5) of the nasal bridge region (1) to the contact point (5) of the base region (3) in the plane of symmetry X-X of the mask between the nasal bridge region and upper lip region, and the width (B2).

8. The mask bead according to at least one of the preceding claims, **characterized in that** the height (H1) in the nasal bridge region measured from the contact point (5) of the mask body to the contact region (4) is always equal to or greater than the height (H2) in the lateral region measured from the contact point (5) of the mask body to the contact region (4).

9. The mask bead for a patient interface according to at least one of the preceding claims, **characterized in that** the contact region (4) at the transitions of the nasal bridge region (6) to the lateral region (2) and the base region (3) to the lateral region (2) has corner points (E1-E4) that serve to align the mask on the face.

10. The mask bead according to claim 9, **characterized in that** the bottom corner points (E3, E4) are located on both sides of the plane of symmetry (x-x) and serve to align the bead on the nasolabial fold.

11. The mask bead according to at least one of claims 9 or 10, **characterized in that** the top corner points (E1, E2) lie on the side of the nose in the region, and define the end of the support region, and hence the transition to the nasal bridge region with the width (B1).

12. The mask bead according to at least one of the preceding claims, **characterized in that** the material of the mask bead has an elasticity that lies above 800%.

## Revendications

1. Jupe de masque pour une interface patient comprenant une zone de contact, une zone du dos du nez (1), des zones latérales (2), une zone de base (3) et une zone d'appui (4) sur le visage du patient, dans laquelle la zone d'appui (4) s'étend en partant des zones latérales (2) et de la zone de base (3) ainsi que de la zone du dos du nez (1) vers une ouverture (6), dans laquelle la zone d'appui (4) est exécutée sous la forme d'une lèvre d'étanchéité, laquelle borde l'ouverture centrale (6), qui sert au moins à l'insertion du nez du patient, dans laquelle la zone du dos du nez (1) présente une largeur (B1) au niveau de la zone de transition entre la zone du dos du nez (1) et la zone latérale (2), la jupe de masque présente une largeur maximale (B2) et le rapport des largeurs (B1) à (B2) est de un pour trois ou supérieur et dans lequel une épaisseur de paroi (W1) s'élève au maximum à 0,6 mm dans la zone du dos du nez (1), **caractérisée en ce que** la zone de base (3), qui présente une épaisseur de paroi (W3), est aussi finement exécutée contrairement aux zones latérales (2), qui présentent une épaisseur de paroi (W2), dans laquelle la zone de base (3) présente une épaisseur de paroi (W3) se situant dans la plage 0,8 - 1,2 mm et/ou la zone latérale (2) présente une épaisseur de paroi (W2) se situant dans la plage 1,5 - 2,8 mm, et dans laquelle la zone d'appui (4) présente une épaisseur de paroi quasiment constante (W4) se situant dans la plage 0,2 - 0,75 mm.

2. Jupe de masque selon la revendication 1, **caractérisée en ce que** la zone du dos du nez (1) présente une largeur (B1) se situant dans la plage 27 - 37 mm.

3. Jupe de masque selon l'une au moins des revendications précédentes, **caractérisée en ce que** la hauteur (H1) dans la zone du dos du nez mesurée du point de contact (5) avec la coque du masque jusqu'à la zone d'appui (4) se situe dans la plage 18 - 27 mm.

4. Jupe de masque selon l'une au moins des revendications précédentes, **caractérisée en ce que** la hauteur (H4) dans la zone du dos du nez mesurée du point de contact (5) avec la coque du masque jusqu'à la transition entre la zone du dos du nez (1) et la zone d'appui (4) se situe dans la plage 14 - 23 mm.

5. Jupe de masque selon l'une au moins des revendications précédentes, **caractérisée en ce que** la zone du dos du nez (1) présente un contour incurvé (R).

6. Jupe de masque selon l'une au moins des revendications précédentes, **caractérisée en ce que** l'épaisseur de paroi dans la zone latérale diminue continuellement de la base jusqu'à la zone d'appui (4).

7. Jupe de masque selon l'une au moins des revendications précédentes, **caractérisée en ce que** le rapport de la largeur (B2) et de la longueur (L) de la jupe de masque mesurée du point de contact (5) avec la zone du dos du nez (1) jusqu'au point de contact (5) avec la zone de base (3) dans le plan de symétrie X-X du masque entre la zone du dos du nez et la zone de la lèvre supérieure est d'environ 1 pour 1.

8. Jupe de masque selon l'une au moins des revendications précédentes, **caractérisée en ce que** la hauteur (H1) dans la zone du dos du nez mesurée du point de contact (5) avec la coque du masque jusqu'à la zone d'appui (4) est toujours aussi grande ou plus grande que la hauteur (H2) dans la zone latérale mesurée du point de contact (5) avec la coque du masque jusqu'à la zone d'appui (4).

9. Jupe de masque pour une interface patient selon l'une au moins des revendications précédentes, **caractérisée en ce que** la zone d'appui (4) au niveau des transitions entre la zone du dos du nez (1) et la zone latérale (2), et entre la zone de base (3) et la zone latérale (2) présente des points d'angle (E1 - E4) qui servent à orienter le masque sur le visage.

10. Jupe de masque selon la revendication 9, **caractérisée en ce que** les points d'angle inférieurs (E3, E4) se trouvent de part et d'autre du plan de symétrie (x-x) et servent à orienter la jupe au niveau de la fente naso-labiale.

11. Jupe de masque selon l'une au moins des revendications 9 ou 10, **caractérisée en ce que** les points d'angle supérieurs (E1, E2) se trouvent sur les côtés du nez dans la zone et qu'ils définissent la fin de la zone de support et ainsi la transition vers la zone du dos du nez avec la largeur (B1).

12. Jupe de masque selon l'une au moins des revendications précédentes, **caractérisée en ce que** le matériau de la jupe de masque présente une extensibilité supérieure à 800 %.
